Europäisches Patentamt

European Patent Office

Office européen des brevets

(19)

(11) Numéro de publication : **0 248 964 B1**

# FASCICULE DE BREVET EUROPEEN

(12)

(45) Date de publication du fascicule du brevet :
09.01.91 Bulletin 91/02

(51) Int. Cl.⁵ : **A61F 13/04, A43B 7/00**

(21) Numéro de dépôt : 86401238.0

(22) Date de dépôt : 09.06.86

(54) **Chaussure à appui talonnier et à contacts minimisés au niveau de l'avant-pied, notamment pour usage post-chirurgical ou post-traumatique.**

(43) Date de publication de la demande :
16.12.87 Bulletin 87/51

(45) Mention de la délivrance du brevet :
09.01.91 Bulletin 91/02

(84) Etats contractants désignés :
AT BE CH DE FR GB IT LI LU NL SE

(56) Documents cités :
EP-A- 0 077 713
FR-A- 2 097 294
US-A- 2 725 648
US-A- 3 584 402

(73) Titulaire : **Barouk, Louis Samuel**
**Chemin de la Roche**
**F-33370 Yvrac (FR)**

(72) Inventeur : **Barouk, Louis Samuel**
**Chemin de la Roche**
**F-33370 Yvrac (FR)**

(74) Mandataire : **Thébault, Jean-Louis**
**Cabinet Thébault S.A. 50 Cours de Verdun**
**F-33000 Bordeaux (FR)**

## Description

La présente invention se rapporte à une chaussure, notamment à usage post-chirurgical ou post-traumatique, destinée à être portée par un patient dont l'avant-pied est atteint d'une affection ou d'une gêne quelconque rendant douloureux ou nocif tout contact externe dudit avant-pied, en particulier avec le sol, cette chaussure comprenant une tige qui est solidaire d'un bloc-semelle dont la partie postérieure est constituée par un talon nettement plus épais à l'avant qu'à l'arrière, déterminant ainsi, au niveau de sa partie supérieure, une inclinaison vers le haut et vers l'avant de la chaussure (c.f. par exemple les documents US-A-2, 725,648 et EP-A-77.713).

Les chaussures existantes de ce type peuvent par exemple être portées par des patients dont l'avant-pied a subi une intervention chirurgicale ou est le siège d'un oedème, d'une inflammation, d'une blessure ou d'une autre affection.

En pareils cas, ces chaussures maintiennent en effet le pied en léger talus pour interdire tout contact douloureux de l'avant-pied avec le sol et permettent donc au patient de marcher à peu près normalement, sans l'utilisation contraignante et inconfortable de cannes, béquilles ou plâtres de marche.

Si, les chaussures de ce type sont complètement tronquées à l'avant pour que l'avant-pied s'étende totalement à l'extérieur et sans aucun appui ou contact externe, (c.f. par exemple le document EP-A-77.713), il s'est avéré à l'usage que ceci était à l'origine d'un certain nombre d'inconvénients.

En premier lieu, dans de telles chaussures tronquées, l'avant-pied est exposé à des chocs antérieurs et latéraux qui non seulement entraînent de vives douleurs, mais sont aussi susceptibles, dans le cas d'une utilisation post-chirurgicale de la chaussure, de provoquer une rupture des matériels d'ostéosynthèse. D'autres inconvénients des chaussures, actuellement dans le commerce, sont à voir dans le mauvais positionnement de l'avant-pied, qui peut occasionner, chez certains sujets, des contractures en flexion dorsale ou plantaire des orteils, ainsi que dans la possibilité d'un déséquilibre antérieur en cours de marche, surtout perceptible chez les personnes âgées, et dans les risques d'apparition de douleurs plantaires au contact du bord antérieur de la première de la chaussure ouverte. On a en outre constaté que l'absence totale d'appui au niveau de l'avant-pied était la cause de troubles trophiques.

La présente invention se propose de remédier à tous ces inconvénients et, pour ce faire, elle a pour objet une chaussure du type spécifié en préambule (US-A-2,725,648) qui se caractérise en ce que ladite partie antérieure mince est flexible dans le sens de la hauteur autour d'une zone située sensiblement au niveau de l'interligne de Lisfranc du pied, les dimensions du talon du bloc-semelle et le dégré de flexibilité de la partie antérieure mince étant en outre calculés, en fonction des dimensions du pied à chausser, pour empêcher tout contact de ladite partie antérieure avec le sol en station debout ou au cours de la marche.

De la sorte, la chaussure post-chirurgicale ou post-traumatique, conforme à la présente invention, est dotée, au niveau de l'avant-pied, d'une protection contre les chocs antérieurs et latéraux, tout en conservant les propriétés des chaussures actuelles, c'est-à-dire le maintien en talus du pied, grâce auquel l'avant-pied reste sans aucun contact avec le sol au cours de la marche. Contrairement aux chaussures classiques, le bout du bloc-semelle n'a pas besoin d'être muni d'un patin d'usure.

La partie antérieure mince (correspondant à la cambrure et au patin d'une chaussure classique), qui assure cette protection contre les chocs de par son surdimensionnement longitudinal et transversal, est certes en contact avec la face plantaire de l'avant-pied, mais il s'agit en fait d'un soutien exempt de contraintes génératrices de douleurs, grâce à la flexibilité dans le sens de la hauteur de la partie antérieure et à l'appui au sol exclusivement talonnier qui, ajouté au maintien en talus du pied, réalisé par le talon compensé à pente inversée du bloc-semelle, interdit tout report du poids du corps sur l'avant-pied. Par ailleurs, ce soutien non douloureux de l'avant-pied élimine les risques d'apparition de troubles trophiques et améliore considérablement l'équilibre du patient au cours de la marche.

En complément, le talon du bloc-semelle est compressible et présente un degré de compressibilité augmentant de l'arrière vers l'avant, ce qui autorise une décomposition naturelle des mouvements du pied au cours de la marche.

Avantageusement, la partie avant du talon du bloc-semelle est conformée selon un coin dirigé vers l'avant de la chaussure pour conférer à celle-ci la meilleure assise possible lors des déplacements du patient, assise qui peut être renforcée par un élargissement du talon du bloc-semelle sur les côtés, en direction de sa surface d'appui au sol.

En outre, un dégagement est formé dans le talon, entre sa partie en forme de coin et la partie antérieure mince du bloc semelle, le fond de ce dégagement étant de préférence situé légèrement en avant de la zone de flexion de cette dernière, pour éviter à ce niveau tout blocage ou écrasement de la voûte plantaire, qui serait sinon source de douleurs.

Pour des raisons évidentes de simplification de la fabrication de la chaussure objet de l'invention, le talon et la partie antérieure mince du bloc-semelle peuvent être avantageusement moulés en une seule pièce, la compressibilité croissante du talon résultant dès lors de la forme particulière décrite ci-dessus, de la partie avant de ce dernier. Si cela ne s'avérait pas suffisant, on pourrait former des alvéoles dans le talon du bloc-semelle.

En outre, dans le cadre de la recherche d'une minimisation des appuis douloureux au niveau de l'avant-pied, la partie antérieure du bloc-semelle présente, sur sa face supérieure, un évidement convenablement positionné et dimensionné pour recevoir au moins la saillie inférieure de la première tête métatarsienne du pied, de tels évidements pouvant d'ailleurs être éventuellement prévus pour les autres têtes métatarsiennes du pied.

De préférence, la partie antérieure mince du bloc-semelle sera chanfreinée au niveau de l'arête inférieure de son bord frontal pour, dans le cas où elle subirait une flexion extrême accidentelle, réduire au maximum ses risques de contact avec le sol.

Selon une autre caractéristique importante de l'invention, la tige se prolonge à l'avant de la chaussure en ayant ses parois dressées le long des bords de la partie antérieure du bloc-semelle.

La tige de la chaussure constitue ainsi une protection supplémentaire, qui notamment abrite l'avant-pied vis-à-vis des intempéries, sans pour autant l'enserrer fermement et donc sans le soumettre à des contraintes douloureuses. En effet, la tige prend naissance sur le bord de la partie antérieure mince, c'est-à-dire à bonne distance de l'avant pied, au moins au niveau de la partie de celui-ci qui est susceptible d'être douloureuse.

Avantageusement, la tige comprend deux quartiers en une matière souple, réunis à l'arrière de la chaussure et se dressant respectivement le long d'une partie au moins des bords latéraux du bloc-semelle, ces quartiers pouvant être écartés l'un de l'autre pour permettre l'insertion du pied dans la chaussure, par application directe de la face plantaire du pied sur le fond de cette dernière et pouvant être reliés l'un à l'autre par une ou plusieurs attaches.

Grâce à ces dispositions, le patient peut, sans aucune souffrance, introduire très facilement son pied dans la tige de la chaussure.

Par ailleurs, en prévoyant des attaches ajustables, telles que des bandes auto-accrochantes, des lanières à boucle ou des lacets, pour réunir les deux quartiers de la tige, on dispose d'une possibilité de serrage indépendant de la tige, à chaque niveau du pied (postérieur, médian, antérieur). De la sorte, on peut convenablement serrer la partie postérieure de la tige autour de l'arrière-pied et simultanément réaliser un serrage lâche au niveau de la partie antérieure pour, à l'intérieur de cette dernière, conférer un maximum d'aisance à l'avant-pied susceptible d'être douloureux.

Au surplus, les attaches ajustables permettent, sur des avant-pieds affectés par un oedème, de suivre les variations de grosseur de ce dernier, notamment dans le cas des oedèmes post-chirurgicaux.

Par ailleurs, une languette souple relevable est de préférence fixée, par une liaison formant charnière, le long du bord frontal de la partie antérieure mince du bloc-semelle et s'étend entre les deux quartiers de la tige.

Cette languette peut être rabattue entre les deux quartiers de la tige avant la fermeture des attaches, lesquelles s'appliqueront ensuite sur cette languette, sans venir en contact direct avec l'avant-pied et donc sans risquer de le blesser sous l'effet du serrage qu'elles exercent.

En variante, cette protection de l'avant-pied vis-à-vis de l'action de serrage des attaches peut être obtenue par une languette s'étendant latéralement sur le bord supérieur libre de l'un des quartiers de la tige, au niveau de la zone d'application des attaches.

On sait par ailleurs que les oedèmes que l'on observe après les opérations chirurgicales de l'avant-pied, touchent essentiellement le gros orteil et c'est pourquoi il est prévu, selon une caractéristique additionnelle de la présente invention, que la partie antérieure mince du bloc-semelle de la chaussure qui en fait l'objet soit nettement plus renflée dans sa moitié longitudinale interne que dans sa miitié longitudinale externe. En d'autres termes, le flanc interne de la chaussure est nettement plus important que dans les chaussures classiques.

Enfin, il sera bon en complément que le bord interne de la moitié longitudinale interne renflée de la partie antérieure mince soit sensiblement parallèle à l'axe longitudinal central de cette dernière, pour éviter que le gros orteil ne soit rabattu vers l'intérieur, comme c'est le cas dans les chaussures classiques. Ceci est particulièrement recherché dans les suites opératoires pour Hallux Valgus qui représentent environ 80% des cas d'interventions sur l'avant-pied.

Un mode de réalisation de la chaussure conforme à la présente invention va maintenant être décrit plus en détails, mais uniquement à titre d'exemple non-limitatif, en référence aux dessins annexés dans lesquels :

      – la figure 1 est une vue latérale de cette chaussure ;

      – la figure 2 est une vue en coupe longitudinale de la chaussure de la figure 1, en place autour du pied d'un patient ;

      – les figures 3 et 4 sont respectivement une vue de dessus et une vue de dessous de cette même chaussure ; et

      – la figure 5 est une vue en coupe effectuée selon la ligne V-V de la figure 1.

La chaussure à usage post-chirurgical ou post-traumatique, qui est exemplifiée, se compose, d'une manière connue en soi, d'une tige 1 reposant sur un bloc-semelle 2 dont la partie postérieure est constituée par un talon 3 qui est nettement plus épais à l'avant qu'à l'arrière pour doter la chaussure d'un appui exclusivement talonnier.

Selon la caractéristique première de l'invention, le bloc-semelle 2 comprend en outre une partie antérieure mince 4 qui prolonge vers l'avant la partie supérieure du talon 3, en conservant la même inclinaison que celle-ci vers le haut. Dans ce qui suit, la zone avant 4a et la zone centrale 4b de cette partie antérieure mince 4 seront respectivement dénommées "patin" et "cambrure" par analogie avec les chaussures classiques.

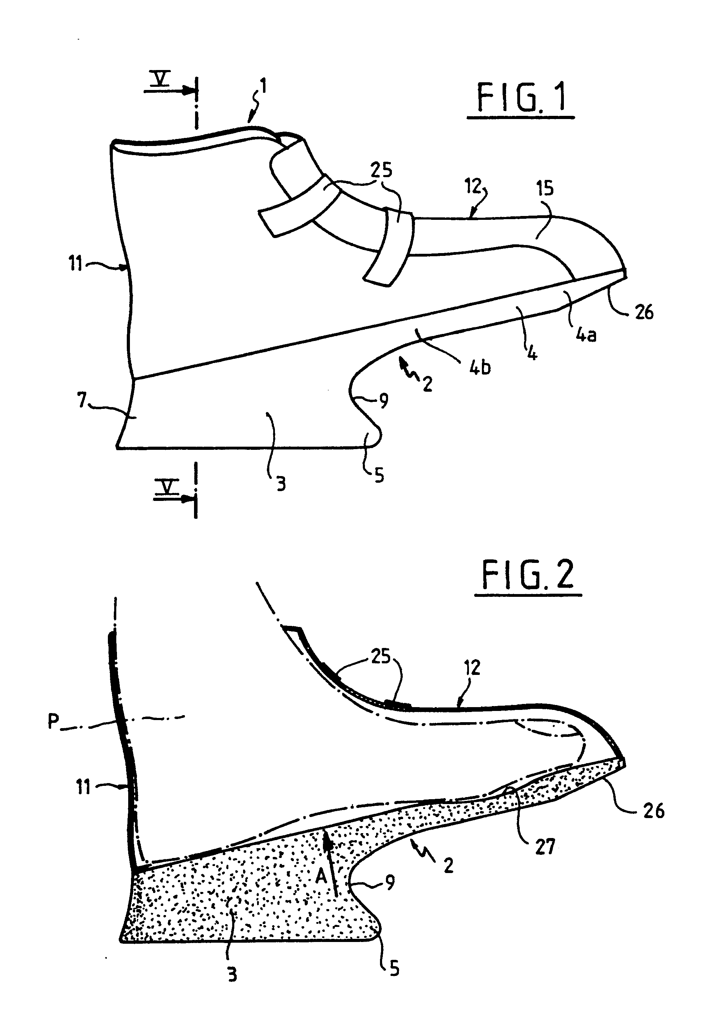

En complément, la partie antérieure mince 4 du bloc-semelle 2 présente, vue en plan, des dimensions supérieures aux dimensions correspondantes du pied et est en outre réalisée en un matériau qui la dotera d'une rigidité suffisante, propre à lui conférer une propriété de quasi indéformabilité dans le sens de la longueur et de la largeur. Par contre, la partie antérieure mince 4 est conçue pour pouvoir fléchir vers le bas, autour d'une zone (flèche A de la figure 2) située sensiblement au niveau de l'interligne de Lisfranc du pied.

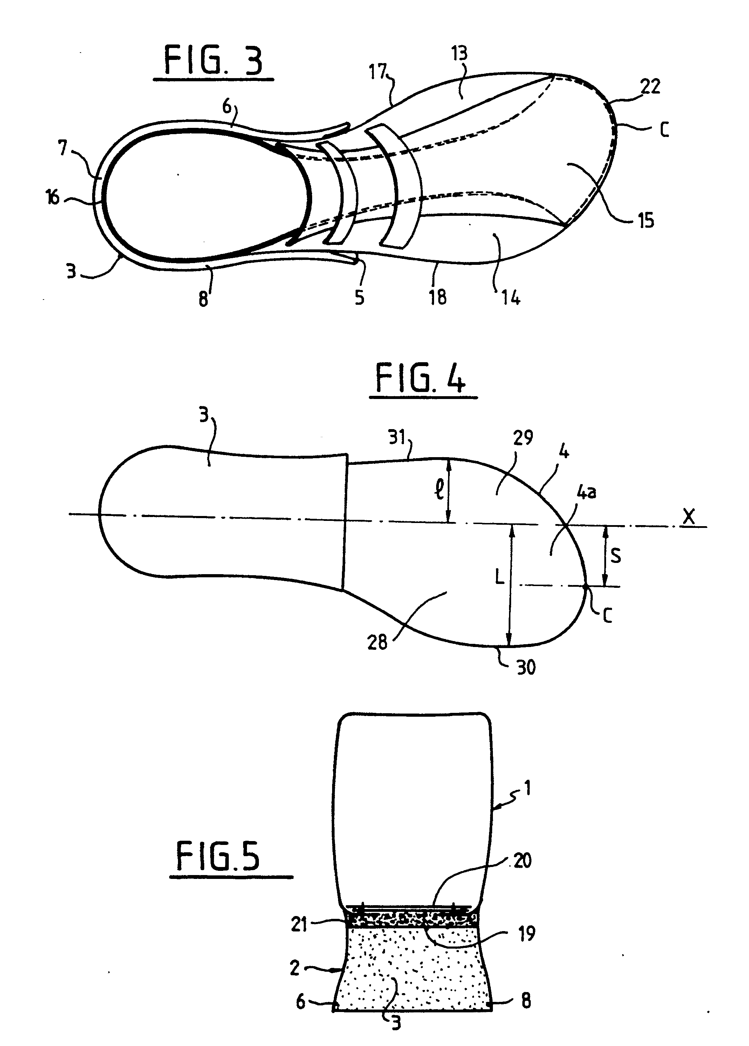

Pour en revenir au talon 3, on précisera qu'il est compressible, avec un dégré de compressibilité allant en augmentant de l'arrière vers l'avant, et on observera encore, sur les figures 1 et 2, que sa partie avant présente la forme d'un coin 5 dirigé vers l'avant de la chaussure, de façon à augmenter au maximum la surface d'appui au sol du bloc-semelle 2. Dans le même but et comme l'illustrent les figures 3 et 5, sous les références numériques 6, 7 et 8, le talon 3 s'élargit légèrement sur les côtés, en direction de sa surface d'appui au sol.

Il est important de noter ici que le coin 5 du talon 3 et la cambrure 4b de la partie antérieure mince 4 délimitent entre eux un dégagement ou une dépouille 9 dont le fond est situé légèrement en avant de la zone de flexion A de cette dernière.

Le bloc-semelle 2 peut être avantageusement confectionné par moulage en une seule pièce d'une matière synthétique possédant des propriétés d'élasticité et de compressibilité ou par collage de plusieurs éléments faits d'une telle matière. La compressibilité variable de son talon 3 résultera alors de la configuration en coin de sa partie avant combinée à la présence du dégagement 9.

La tige 1 de la chaussure exemplifiée comprend, pour sa part, une partie postérieure 11 située dans son ensemble à l'aplomb du talon 3 et, selon une autre caractéristique de l'invention, elle se prolonge à l'avant de la chaussure par une partie antérieure 12 destinée à envelopper, au moins en partie, l'avant-pied du patient sans le soumettre à une quelconque contrainte, la partie postérieure 11 de la tige 1 étant quant à elle dessinée, conformément à la morphologie du pied du patient, pour enserrer l'arrière-pied, qui inclut principalement le tarse et le talon, et remonter sensiblement jusqu'aux malléoles péronéo-tibiales.

Plus précisément, la tige 1 est globalement formée de deux quartiers latéraux 13 et 14 et d'une languette frontale 15, tous trois confectionnés en toile, en tissu épais ou en cuir souple.

Comme on peut le voir sur la figure 3, les deux quartiers 13 et 14 sont réunis à l'arrière de la chaussure par une ligne de couture verticale 16 et se dressent par ailleurs le long des borda latéraux 17, 18 du bloc-semelle 2 (voir figure 3) en s'arrêtant à courte distance du point d'extrémité frontal C du patin 4a.

La figure 5 montre qu'en fait les deux quartiers 13, 14 de la tige 1 sont, par leur bord inférieur, rabattus et cousus sous une première de montage 19 sur laquelle est mise en place une première de confort 20, l'élément de chaussure ainsi formé étant ensuite réuni, avec interposition d'un rembourrage 21, au bloc-semelle 2, par exemple par collage ou par moulage de ce dernier au contact dudit élément de chaussure. La première de montage 19 et la première de confort 20 qui, pour des raisons de clarté du dessin, n'ont pas été représentées sur la figure 2, présentent sensiblement la même largeur que la face supérieure du bloc-semelle 2 et s'étendent sur approximativement toute la longueur de ce dernier.

En revenant à la figure 3, on peut voir que la languette 15 est simplement fixée, par une ligne de couture formant charnière 22, le long de la portion, laissée libre par les deux quartiers 13, 14, du bord frontal du patin 4a de la partie antérieure mince 4. Le patient pourra ainsi relever aisément la languette 15 pour ouvrir la tige 1 et, après avoir écarté légèrement les quartiers 13, 14, mettre en place son pied, sans peine et sans douleur, à l'intérieur de la chaussure, par application directe de la partie plantaire du pied sur la première de confort 20 de cette dernière.

Dès lors, la languette 15 peut être rabattue sur les quartiers 13, 14 et, dans cette position, elle sera maintenue en place par deux pattes auto-accrochantes de type "VELCRO" 25, prévues sur les quartiers 13, 14, à hauteur du cou-de-pied.

Il est à noter ici que ces pattes auto-accrochantes destinées à resserrer la tige 1 autour du pied à chausser, rendent possible un réglage indépendant du serrage de la tige à chaque niveau du pied (postérieur, médian, antérieur), permettant ainsi à la tige de suivre les fluctuations locales de volume du pied, dues par exemple à un oedeme post-chirurgical.

Une fois mis en place dans la chaussure en étant immobilisé à l'arrière, le pied P du patient sera maintenu en léger talus, c'est-à-dire relevé vers le haut, et grâce à la compressibilité croissante du talon 3 du bloc-semelle 2 ajoutée à la flexibilité de la partie antérieure 4 au niveau de l'interligne de Lisfranc, le patient pourra marcher normalement sans que son avant-pied, atteint d'affection douloureuse ou venant d'être opéré, ne vienne en contact avec le sol. Bien entendu, pour ce faire, l'amplitude de débattement maximum du patin 4a de la partie antérieure mince 4 devra être convenablement ajustée par un dimensionnement approprié de la hauteur frontale du talon 3 et de l'épaisseur de la partie antérieure mince 4. En complément, l'arête inférieure du bord frontal du patin 4a sera chanfreinée, comme représenté en 26 sur les figures 1 et 2, pour minimiser les risques de contact avec le sol dans le cas d'une flexion excessive accidentelle.

Au cours de la marche qui pourra se faire avec un déroulement naturel du pied, le patient conservera un bon équilibre grâce à la surface d'appui au sol élargie en 5, 6, 7 et 8 du bloc-semelle 2 de sa chaussure. Au surplus, tout écrasement douloureux de la voûte plantaire lui sera évité au niveau de la zone de flexion A de la partie antérieure 4 du bloc-semelle 2, grâce au dégagement 9.

Le surdimensionnement de la partie antérieure mince 4 protègera en outre l'avant-pied contre les chocs latéraux ou frontaux qui seraient susceptibles de lui infliger de vives douleurs. Par ailleurs, bien qu'étant en contact avec cette partie antérieure 4 et complètement entouré par la tige 1, qui constitue une protection supplémentaire, l'avant-pied ne sera soumis à aucune contrainte douloureuse de la part de ces dernières, tant par le fait que la tige 1 l'enveloppe avec une certaine ampleur que par sa "mise en décharge" résultant de l'appui exclusivement talonnier, procuré par le talon 3 du bloc-semelle 2, ainsi que du resserrement de l'arrière-pied, assuré par la partie postérieure 11 de la tige 1 et les pattes auto-accrochantes 25. Dans le cadre de cette minimisation des appuis douloureux au niveau de l'avant-pied, la face supérieure du patin 4a de la partie antérieure 4 du bloc-semelle 2 sera avantageusement pourvue d'un évidement 27 convenablement positionné et dimensionné pour recevoir la saillie inférieure de la première tête métatarsienne et éventuellement celles des autres têtes métatarsiennes.

Il est encore à noter que le soutien non générateur de douleurs de l'avant-pied confère au patient un bon équilibre au cours de la marche et prévient additionnellement les troubles trophiques.

En se référant à la figure 4, on va maintenant donner quelques précisions au sujet du dimensionnement du patin 4a de la partie antérieure mince 4 du bloc-semelle 2.

Comme on le sait, les interventions chirurgicales effectuées sur l'avant-pied, suivies de la formation d'un oedème, concernent essentiellement le gros orteil. Celui-ci est donc bien souvent augmenté de volume et peut même porter une broche d'ostéosynthèse saillant vers l'avant.

C'est pourquoi, dans la chaussure objet de la présente invention, le flanc interne 28 du patin 4a, qui est appelé à soutenir le gros orteil du pied, est bien plus surdimensionné ou renflé, à l'avant et sur le côté, que le flanc externe 29. Sur la figure 4, cela se traduit par le fait que, d'une part, la distance L entre le bord interne 30 du patin 4a et l'axe longitudinal central X de la partie antérieure mince 4a est nettement supérieure à la distance I qui sépare ce même axe X du bord externe 31 du potin 4a et que, d'autre part, le point d'extrémité frontal C de ce dernier est désaxé vers l'intérieur de l'axe X d'une distance S.

Au surplus, le gros orteil opéré ou affecté par un oedème doit conserver son axe sensiblement parallèle à celui du pied. Pour satisfaire cette exigence, le bord interne 30 du patin 4a de la partie antérieure 4 du bloc-semelle de la chaussure selon la présente invention est prévu sensiblement parallèle à l'axe X de cette dernière.

Bien entendu, diverses adaptations ou modifications, restant dans le cadre de la présente invention, peuvent être apportées à la chaussure qui vient d'être décrite. C'est ainsi notamment que les pattes auto-accrochantes 25 peuvent être remplacées par des lanières à boucles de fermeture ou des lacets qui, permettent aussi des réglages indépendants du serrage de la tige aux différents niveaux du pied. Par ailleurs, la tige 1 peut être conçue sans la languette frontale 15 et, dans ce cas, l'un des deux quartiers 13 ou 14 sera muni d'une languette de protection latérale sur laquelle les pattes auto-accrochantes 25 ou les lanières ou lacets équivalents s'appliqueront, le cou-de-pied étant ainsi protégé contre les effets du resserrement produit par ces derniers.

Il va de soi aussi que les dimensions de la chaussure selon l'invention seront fonction de celles du pied à chausser. Pour fixer les idées, on précisera que dans une chaussure de pointure 37-38, le bloc-semelle 2 aura une longueur totale de 245 mm au niveau de sa face supérieure, une hauteur de 30 mm à l'arrière et une hauteur de 58 mm à l'avant dans le plan de l'extrémité du coin 5.

Enfin, on ajoutera encore que la chaussure exemplifiée est une chaussure de droite et qu'une chaussure de gauche, conçue selon les principes de l'invention, présentera vue en plan une forme symétrique à celle de la chaussure de droite.

## Revendications

1. Chaussure, notamment à usage post-chirurgical ou post-traumatique, destinée à être portée par un patient dont l'avant-pied est atteint d'une affection ou d'une gêne quelconque rendant douloureux et nocif tout contact externe dudit avant-pied, en particulier avec le sol, cette chaussure comprenant une tige (1) qui est solidaire d'un bloc-semelle (2) dont la partie postérieure est constituée par un talon (3) nettement plus épais à l'avant qu'à l'arrière déterminant ainsi, au niveau de sa partie supérieure, une inclination vers le haut et vers l'avant de la chaussure, caractérisée en ce que le bloc-semelle (2) comprenant en outre une partie antérieure mince (4) qui prolonge vers l'avant la partie supérieure du talon en conservant la même inclinaison que celle-ci, ladite partie antérieure mince présentant des dimensions transversale et longitudinale supérieures aux dimensions correspondantes du pied (P) et étant pratiquemment indéformable dans le sens de sa longueur et de sa largeur, caractérisé en ce que ladite partie antérieure mince (4) est flexible dans le sens de la hauteur autour d'une zone (A) située sensiblement au niveau de l'interligne de Lisfranc du pied et les dimensions du talon (3) du bloc-semelle (2) et le degré de flexibilité de la partie antérieure mince (4) sont calculés, en fonction des dimensions du pied à chausser, pour empêcher tout contact de ladite partie antérieure (4) avec le sol en station debout ou au cours de la marche.

2. Chaussure selon la revendication 1, caractérisée en ce que le talon (3) du bloc-semelle (2) est compressible et présente un degré de compressibilité augmentant de l'arrière vers l'avant.

3. Chaussure selon la revendication 1 ou 2, caractérisée en ce que la partie avant du talon (3) du bloc-semelle (2) est conformée selon un coin (5) dirigé vers l'avant de la chaussure.

4. Chaussure selon l'une quelconque des revendications 1 à 3, caractérisée en ce que le talon (3) du bloc-semelle (2) s'élargit sur les côtés (en 6, 7 et 8) en direction de sa surface d'appui au sol.

5. Chaussure selon la revendication 3 ou 4, caractérisée en ce qu'un dégagement (9) est formé dans le talon (3), entre sa partie en forme de coin (5) et la partie antérieure mince (4) du bloc-semelle (2), le fond de ce dégagement étant situé légérement en avant de la zone de flexion (A) de cette dernière.

6. Chaussure selon l'une quelconque des revendications 1 à 5, caractérisée en ce que la partie antérieure mince (4) du bloc-semelle (2) présente, sur sa face supérieure, un évidement (27) convenablement positionné et dimensionné pour recevoir au moins la saillie inférieure de la première tête métatarsienne du pied (P).

7. Chaussure selon l'une quelconque des revendications 1 à 6, caractérisée en ce que la partie antérieure mince (4) du bloc-semelle (2) est chanfreinée (en 26) au niveau de l'arête inférieure de son bord frontal.

8. Chaussure selon l'une quelconque des revendications 1 à 7, caractérisée en ce que, dans sa moitié longitudinale interne (28), la partie antérieure mince (4) est nettement plus renflée que dans sa moitié longitudinale externe (29).

9. Chaussure selon la revendication 8, caractérisée en ce que le bord interne de la moitié longitudinale interne renflée (28) de la partie antérieure (4) est sensiblement parallèle à l'axe longitudinal (X) de cette dernière.

10. Chaussure selon l'une quelconque des revendications 1 à 9, caractérisée en ce que la tige (1) se prolonge à l'avant de la chaussure, avec ses parois se dressant le long des bords de la partie antérieure (4) du bloc-semelle (2).

11. Chaussure selon la revendication 10, caractérisée en ce que la tige (1) comprend deux quartiers (13, 14) en une matière souple, réunis à l'arrière de la chaussure et se dressant respectivement le long d'une partie au moins des bords latéraux (17, 18) du bloc-semelle (2), ces quartiers pouvant être écartés l'un de l'autre pour permettre l'insertion du pied dans la chaussure, par application directe de la face plantaire du pied sur le fond de cette dernière et pouvant être reliés l'un à l'autre par une ou plusieurs attaches (25).

12. Chaussure selon la revendication 11, caractérisée en ce qu'une languette souple relevable (15) est fixée, par une liaison formant charnière (22), le long du bord frontal de la partie antérieure mince (4) du bloc-semelle (2) et s'étend entre les deux quartiers (13, 14) de la tige (1).

13. Chaussure selon la revendication 12, caractérisée en ce que l'un des quartiers (13, 14) comporte une languette de protection s'étendant latéralement sur le bord supérieur libre de l'un des quartiers (13 ou 14) de la tige (1), au niveau de la zone d'application des attaches.

## Ansprüche

1. Schuh, insbesondere zum Gebrauch nach einer Operation oder Verletzung, bestimmt zum Tragen durch einen Patienten, dessen Vorderfuß von einem krankhaften oder irgendeinem Zustand befallen ist, bei dem jeder äußere kontakt des besagten Vorderfußes insbesondere mit dem Boden schmerzhaft und schädlich ist, wobei dieser Schuh einen Schaft (1) umfaßt, der fest mit einem Sohlenblock (2) verbunden ist, dessen hinterer Teil durch einen Absatz (3) gebildet wird, der vorne stärker als hinten ist, um auf diese Weise in Höhe seiner Oberpartie eine nach oben und vorne gerichtete Neigung des Schuhs zu liefern, wobei der Sohlenblock (2) zudem einen dünnen vorderen Abschnitt (4) umfaßt, der die obere Partie des Absatzes nach vorne verlängert, indem er die gleiche Neigung wie diese beibehält, wobei der besagte dünne vordere Abschnitt Quer- und Längsabmessungen größer als die korrespondierenden Abmessungen des Fußes (P) aufweist und praktisch in Richtung seiner Länge und seiner Breite undeformierbar ist, dadurch **gekennzeichnet**, daß der besagte dünne vordere Abschnitt (4) in Höhenrichtung um eine Zone (A), die sich etwa auf dem Niveau der Trennlinie zwischen Fußwurzel und Mittelfuß befindet, flexibel ist und die Abmessungen des Absatzes (3) des Sohlenblocks (2) und der Flexibilitätsgrad der dünnen vorderen Abschnitts (4) asl Funktion von Abmessungen des zu beschuhenden Fußes berechnet sind, um jeden Kontakt des besagten vorderen Abschnitts (4) mit dem Boden beim Stehen oder Gehen zu verhindern.

2. Schuh gemäß Anspruch 1, dadurch gekennzeichnet, daß der Absatz (3) des Sohlenblocks (2) kompressibel ist und einen von hinten nach vorne steigenden Kompressibilitätsgrad aufweist.

3. Schuh gemäß Anspruch 1 oder 2, dadurch gekennzeichnet, daß der vordere Abschnitt des Absatzes (3) des Sohlenblocks (2) gemäß einem bezüglich des Schuhs nach vorne gerichteten Keil (5) ausgebildet ist.

4. Schuh gemäß einem der Ansprüche 1 bis 3, dadurch gekennzeichnet, daß der Absatz (3) des Sohlenblocks (2) zu den Seiten hin (bei 6, 7 und 8) in Richtung seiner Abstützfläche auf den Boden verbreitert ist.

5. Schuh gemäß Anspruch 3 oder 4, dadurch gekennzeichnet, daß eine Hinterschneidung (9) in dem Absatz (3) zwischen seinem keilförmigen Abschnitt (5) und dem dünnen vorderen Abschnitt (4) des Sohlenblocks (2) ausgebildet ist, wobei der Boden dieser Hinterschneidung etwas vor dem biegebereich (A) des letzteren angeordnet ist.

6. Schuh gemäß einem der Ansprüche 1 bis 5, dadurch gekennzeichnet, daß der dünne vordere Abschnitt (4) des Sohlenblocks (2) an seiner Oberseite eine Ausnehmung (27) aufweist, die passend positioniert und dimensioniert ist, um wenigstens den unteren ballen des Kopfes des ersten Mittelfußknochens des Fußes (P) aufzunehmen.

7. Schuh gemäß einem der Ansprüche 1 bis 6, dadurch gekennzeichnet, daß er dünne vordere Abschnitt (4) des Sohlenblocks (2) (bei 26) auf dem Niveau der Unterkante seines Vorderrandes abgeschrägt ist.

8. Schuh gemäß einem der Ansprüche 1 bis 7, dadurch gekennzeichnet, daß der dünne vordere Abschnitt (4) in seiner inneren Längshälfte (28) deutlich mehr gewölbt als in seiner äußeren Längshälfte (29) ist.

9. Schuh gemäß Anspruch 8, dadurch gekennzeichnet, daß der Innenrand der gewölbten inneren Längshälfte (28) des vorderen Abschnitts (4) etwa parallel zur Längsachse (X) dieses letzteren verläuft.

10. Schuh gemäß einem der Ansprüche 1 bis 9, dadurch gekennzeichnet, daß der Schaft (1) sich bezüglich des Schuhs nach vorne mit seinen Wänden sich längs der Ränder des vorderen Abschnitts (4) des Sohlenblocks (2) erhebend verlängert.

11. Schuh gemäß Anspruch 10, dadurch gekennzeichnet, daß der Schaft (1) zwei Stücke (13, 14) aus einem weichen Material umfaßt, die an der Rückseite des Schuhs verbunden sind und sich entsprechend längs eines Teils wenigstens der Seitenränder (17, 18) des Sohlenblocks (2) erheben, wobei diese Stücke voneinander entfernt werden können, um das Einführen des Fußes in den Schuh durch direktes Aufsetzen der Fußsohle auf den boden des letzteren zu ermöglichen, und miteinander durch ein oder mehrere bänder (25) verbunden werden können.

12. Schuh gemäß Anspruch 11, dadurch gekennzeichnet, daß eine anhebbare, weiche Zunge (15) über eine ein Gelenk bildende Verbindung (22) längs der Vorderkante des dünnen vorderen Abschnitts (4) des Sohlenblocks (2) befestigt ist und sich zwischen den beiden Stücken (13, 14) des Schaftes (1) erstreckt.

13. Schuh gemäß Anspruch 12, dadurch gekennzeichnet, daß eines der Stücke (13, 14) eine Schutzzunge umfaßt, die sich seitlich längs des freien Oberrandes des einen der Stücke (13 oder 14) des Schaftes (1) in Höhe des Anbringbereichs von bändern erstreckt.

**Claims**

1. A shoe, particularly for use after surgery or trauma, intended to be worn by a patient whose forefoot is subject to any disorder or discomfort making any external contact of this forefoot, especially with the ground, painful and harmful, which shoe comprises an upper (1) rigid with a block sole (2) whose rear portion is formed by a heel (3) which is substantially thicker at the front than at the rear thereby causing an upward and forward inclination of the upper portion of the shoe, the block sole (2) further comprising a thin front portion (4) which extends the upper portion of the heel forwardly while retaining the same inclination as the latter, this thin front portion having transverse and longitudinal dimensions greater than the corresponding dimensions of the foot (P) and in practice being non-deformable in the direction of its width and its length, characterized in that the thin front portion (4) is flexible in the direction of its height about a zone (A) located substantially at the level of the Lisfranc joint of the foot and in that the dimensions of the heel (3) of the block sole (2) and the degree of flexibility of the thin front portion (4) are calculated as a function of the dimensions of the foot to be shod so as to prevent any contact between this front portion (4) and the ground when in the standing position or during walking.

2. A shoe as claimed in claim 1, characterized in that the heel (3) of the block sole (2) can be compressed and has a degree of compressibility increasing from the rear towards the front.

3. A shoe as claimed in claim 1 or 2, characterized in that the front portion of the heel (3) of the block sole (2) is shaped in the form of a wedge (5) facing the front of the shoe.

4. A shoe as claimed in any one of claims 1 to 3, characterized in that the heel (3) of the block sole (2) broadens out at the sides (at 6, 7 and 8) in the direction of its surface bearing on the ground.

5. A shoe as claimed in claim 3 or 4, characterized in that a recess (9) is formed in the heel (3) between its wedgeshaped portion (5) and the thin front portion (4) of the block sole (2), the base of this recess being located slightly in front of the bending zone (A) of the latter.

6. A shoe as claimed in any one of claims 1 to 5, characterized in that the upper surface of the thin front portion (4) of the block sole (2) has a recess (27) which is suitably positioned and dimensioned to receive at least the lower projection of the first metatarsal head of the foot (P).

7. A shoe as claimed in any one of claims 1 to 6, characterized in that the thin front portion (4) of the block sole (2) is bevelled (at 26) at the level of the lower border of its front edge.

8. A shoe as claimed in any one of claims 1 to 7, characterized in that the inner longitudinal half (28) of the thin front portion (4) is substantially broader than its outer longitudinal half (29).

9. A shoe as claimed in claim 8, characterized in that the inner edge of the broader inner longitudinal half (28) of the front portion (4) is substantially parallel to the longitudinal axis (X) of the latter.

10. A shoe as claimed in any one of claims 1 to 9, characterized in that the upper (1) extends to the front of the shoe, with its walls rising along the edges of the front portion (4) of the block sole (2).

11. A shoe as claimed in claim 10, characterized in that the upper (1) comprises two quarters (13, 14) of flexible material joined at the rear of the shoe and rising respectively along at least one portion of the lateral edges (17, 18) of the block sole (2), which quarters may be spaced from one another to allow the foot to be inserted into the shoe by directly applying the plantar surface of the foot to the base of the latter and may be joined to one another by one or a plurality of connections.

12. A shoe as claimed in claim 11, characterized in that a flexible tongue (15) which can be raised is fastened, by a connection forming a hinge (22), along the front edge of the thin front portion (4) of the block sole (2) and extends between the two quarters (13, 14) of the upper (1).

13. A shoe as claimed in claim 12, characterized in that one of the quarters (13, 14) comprises a protection tongue extending laterally over the free upper edge of one of the quarters (13 or 14) of the upper (1) at the location of application of the connections.

FIG.1

FIG.2

## FIG. 3

## FIG. 4

## FIG.5